# EUROPÄISCHE PATENTSCHRIFT

(11) **EP 2 684 955 B1**
(45) Veröffentlichungstag und Bekanntmachung des Hinweises auf die Patenterteilung: **06.09.2017**
(21) Anmeldenummer: 13179144.4
(22) Anmeldetag: 30.10.2008
(51) Int. Cl.: C12N 15/113, A61K 31/7088

(54) **Verfahren zur Förderung der Angiogenese, Vaskularisierung oder Gefäßreparatur oder zur Hemmung der Tumorangiogenese**
Method for promoting angiogenesis, vascularisation or vessel repair or for inhibiting tumour angiogenesis
Procédé pour stimuler l'angiogenèse, la vascularisation ou la réparation vasculaire ou pour empêcher l'angiogenèse tumorale

(30) Priorität: 30.10.2007 DE 102007052114
(43) Veröffentlichungstag der Anmeldung: 15.01.2014
(62) Teilanmeldung aus: 08844273.6
(73) Patentinhaber: t2cure GmbH, 60596 Frankfurt (DE)
(72) Erfinder: Dimmeler, Stefanie, 60594 Frankfurt (DE); Urbich, Carmen, 40211 Düsseldorf (DE); Zeiher, Andreas, 60594 Frankfurt (DE); Bonauer, Angelika, 60316 Frankfurt (DE)
(74) Vertreter: Krauss, Jan

(56) Entgegenhaltungen:
- WO-A-2008/014008
- DEWS MICHAEL ET AL: "Augmentation of tumor angiogenesis by a Myc-activated microRNA cluster.", NATURE GENETICS SEP 2006, Bd. 38, Nr. 9, September 2006 (2006-09), Seiten 1060-1065, XP009113009, ISSN: 1061-4036
- VENTURINI LETIZIA ET AL: "Expression of the miR-17-92 polycistron in chronic myeloid leukemia (CML) CD34+ cells.", BLOOD 15 MAY 2007, Bd. 109, Nr. 10, 15. Mai 2007 (2007-05-15) , Seiten 4399-4405, XP002518185, ISSN: 0006-4971
- DATABASE EMBL 10. Mai 2004 (2004-05-10), XP002339404, Database accession no. AB176708
- KUEHBACHER ANGELIKA ET AL: "Role of Dicer and Drosha for endothelial microRNA expression and angiogenesis.", CIRCULATION RESEARCH 6 JUL 2007, Bd. 101, Nr. 1, 6. Juli 2007 (2007-07-06), Seiten 59-68, XP002518186, ISSN: 1524-4571
- KUEHBACHER ET AL: "Targeting microRNA expression to regulate angiogenesis", TRENDS IN PHARMACOLOGICAL SCIENCES, ELSEVIER, HAYWARTH, GB, Bd. 29, Nr. 1, 18. Dezember 2007 (2007-12-18), Seiten 12-15, XP022410624, ISSN: 0165-6147

## Beschreibung

Die Erfindung wird durch die beiliegenden Patentansprüche definiert. Offenbart wird ein Verfahren zur Beeinflussung der miR-92-Expression in einer Zelle, insbesondere zur Förderung der Angiogenese, Vaskularisierung oder Gefäßreparatur oder zur Hemmung oder Blockade der Tumorangiogenese, sowie die Verwendung eines derartigen Verfahrens zur Therapie einer Krankheit oder eines Zustands. Weiterhin betrifft die Offenbarung eine pharmazeutische Zusammensetzung zur Förderung der Angiogenese, Vaskularisierung oder Gefäßreparatur oder zur Hemmung oder Blockade der Tumorangiogenese in einer Zelle.

Das Endothelium spielt eine bedeutende Rolle bei der Erhaltung der Integrität und Funktionalität der Gefäße. Das Wachstum neuer Gefäße geschieht im Erwachsenen durch Arteriogenese, Angiogenese oder Vaskulogenese. Während Arteriogenese als das Wachstum kollateraler Gefäße definiert ist, wird unter Angiogenese das Wachstum neuer Blutgefäße aus Vorexistierenden Gefäßen verstanden. Während der Angiogenese werden ruhende Endothelzellen durch angiogene Faktoren aktiviert und beginnen zu migrieren, proliferieren und organisieren sich in tubulären Strukturen (2). Der Begriff Vaskulogenese beschrieb ursprünglich die Denovoblutgefäßbildung im Embryo aus Angioblasten, bezieht sich aber inzwischen auch auf die Bildung von Blutgefäßen aus endothelialen Vorläuferzellen oder anderen adulten Stammzellen (1). Angiogenese und Vaskulogenese stellen physiologische Entwicklungsprozesse dar, die in der Wiederherstellung des Blutflusses in ischämischen Geweben eine essentielle Rolle spielen und einen grundlegenden Schritt im Wachstum von Tumoren darstellen. Die Förderung der Angiogenese und Neovaskularisierung wurde beispielsweise in von Ischämien betroffenen Patienten als mögliche therapeutische Strategie identifiziert. In der Tumorangiogenese führt die Eindämmung dieser Prozesse zur Repression des Tumorwachstums.

MikroRNAs (miRNAs) sind kleine nicht kodierende RNAs, die die Genexpression auf der posttranskriptionalen Ebene durch Degradation der Ziel mRNA oder durch translationale Repression regulieren (3). Im Gegensatz zu kleinen interferierenden RNAs (siRNA), die an komplementäre mRNA Sequenzen binden, findet die Bindung der miRNA an ein Ziel nicht nur an eine komplementäre RNA statt, sondern bildet komplexere RNA-RNA-Strukturen, die thermodynamisch bevorzugt sind (4). Diese "inkomplette" Bindung erlaubt es, das ein miRNA Molekül an verschiedene mRNA Moleküle binden kann. Die Regulation eines Sets von Genen (im Gegensatz zur Monotherapie mittels eines Gens oder eines Wachstumsfaktors) kann einen Vorteil darstellen, wenn komplexe regulatorische Prozesse dadurch beeinflusst werden können. Bisher sind mehr als 400 miRNAs im menschlichen Genom identifiziert worden, aber die Relevanz der meisten dieser miRNAs für die zelluläre Funktion in physiologischen und pathologischen Prozessen ist nach wie vor unklar. Während es im Stand der Technik beschrieben wurde, dass die Herunterregulation der miRNA-prozessierenden Enzyme Dicer und Drosha die Angiogenese beeinträchtigt (5 - 7), sind nur wenige spezifische miRNAs beschrieben worden, die endotheliale Zellfunktionen und Angiogenese beeinflussen. miR-221 und miR-222 blockieren die endotheliale Zellmigration, Proliferation und Angiogenese *in vitro* durch Interaktion mit dem Stammzellfaktorrezeptor c-kit und Regulierung der eNOS Expression auf indirekte Weise (6, 8). Im Gegensatz dazu trägt die Expression von let7-f und miR-27b zur *in vitro* Angiogenese bei (7).

Im Stand der Technik wurde dem miRNA Cluster miR-17-92 eine starke die Tumorangiogenese fördernde Aktivität zugesprochen. Der miR-17-92 Cluster besteht aus miR-17-5p, miR-17-3p, miR-18a, miR-19a, miR-20a, miR-19b und miR-92-1 (9). Dieser miR-17-92 Cluster ist in Myc-induzierten Tumoren hochreguliert und die einzelnen miRNAs miR-18 und miR-19 konnten als Moleküle identifiziert werden, die mit der Expression antiangiogener Proteine spezifisch interagieren. Eine spezifische Evaluierung der Ziele dieser miRNAs zeigte, dass miR-18 bevorzugt die Expression des konvektiven Gewebewachstumsfaktors (CTGF) supprimiert, während miR-19 mit dem starken Angiogenese-Inhibitor Thrombospondin-1 (TSP-1) interagiert. (10).

Überraschenderweise wurde erfindungsgemäß gefunden, dass miR-92 nicht wie im Stand der Technik beschrieben die Angiogenese fördert, sondern die Migration und Tubusformierung endothelialer Zellen *in vitro* und die Neovaskularisierung *in vivo* stark vermindert. Die Inhibierung von miR-92 erhöht die Neovaskularisierung. Demzufolge besitzt miR-92 eine antiangiogene Aktivität und nicht wie bisher im Stand der Technik beschrieben eine proangiogene Aktivität.

Diese antiangiogene Aktivität steht im Zusammenhang mit der Inhibierung von Schlüsselproteinen, die Angiogenese und endotheliale Aktivität kontrollieren, einschließlich der endothelialen Stickoxidsynthase (eNOS) und Sirtuin1 (SIRT1), die beide für postnatale endotheliale Zellfunktionen essentiell sind (11 bis 13), und Integrin a5, das die endotheliale Zellmotilität und die Interaktion mit der Matrix kontrolliert (14). Die Inhibierung von miR-92 durch miR-92 Inhibitoren stellt eine neue therapeutische Strategie zur Verbesserung der endothelialen Zellfunktion und der Neovaskularisierung dar. Gleichzeitig wurde gefunden, dass die Überexpression von miR-92 Neovaskularisierung vermindert.

### Beschreibung der Erfindung

Die Erfindung beruht darauf, dass miR-92 eine antiangiogene biologische Aktivität entfaltet und nicht wie bisher im Stand der Technik beschrieben, eine proangiogene Aktivität. Die Erfindung wird durch die beiliegenden unabhängigen Patentansprüche 1 und 6 definiert. Besondere Ausführungsformen der Erfindung werden in den Unteransprüchen genannt.

Offenbart wird ein Verfahren zur Beeinflussung der miR-92 Expression in einer Zelle, insbesondere im Rahmen der Angiogenese und/oder der Vaskulogenese. Erfindungsgemäß weist das Verfahren die folgenden Schritte auf:
a) Bereitstellen einer Zelle; und
b1) Verringern der miR-92 Expression in der Zelle zur Förderung der Angiogenese, Vaskularisierung und/oder der Gefäßreparatur durch Einbringen eines antisense Moleküls gegen miR-92 in die Zelle, oder
b2) Erhöhen der miR-92 Expression in der Zelle zur Hemmung der Tumorangiogenese durch Einbringen eines Konstrukts in die Zelle, wobei das Konstrukt eine exprimierbare miR-92 Sequenz aufweist. Ebenso ist es möglich, miR-92 in die Zelle einzubringen.
In einer bevorzugten Ausführungsform ist das Verfahren ein *in vitro* Verfahren.

Der Begriff Verringern oder Erhöhen der miR-92 Expression in der Zelle beruht auf einem Vergleich mit der miR-92 Expression in einer Wildtyp Zelle, wie sie etwa mittels RT-PCR oder Real-time PCR durchgeführt werden kann. Die jeweilige Änderung der Expression kann auch über die funktionalen Auswirkungen der geänderten Expressionsstärke von miR-92 auf die Eigenschaften der Zelle bestimmt werden.

Ein antisense Molekül ist ein einzelsträngiges Molekül, das eine zu einer RNA (hier miR-92) im Wesentlichen revers komplementäre Sequenz aufweist und die biologische Funktion von miR-92 durch Hybridisierung mit der miR-92 inhibiert. Bevorzugter Weise ist das antisense Molekül eine antisense RNA, die optional chemische Modifikationen aufweisen kann.

Das Konstrukt kann ein Plasmid, ein Cosmid, ein Virus oder eine Vorläufer miRNA sein, wobei das Konstrukt vorteilhafter Weise ein Mittel zur Transkription der enthaltenen exprimierbaren miR-92 Sequenz aufweist, wie beispielsweise einen Promoter, der funktionell mit der miR-92 Sequenz verbunden ist.

Unter den hier verwendeten Begriff "miR-92" fallen sowohl Vorläufermoleküle wie pre-92a-1, pre-92a-2 und pre-92b, als auch prozessierte Moleküle wie miR-92a, und miR-92b (siehe auch die Sequenzen gemäß den SEQ ID NO 1 bis 5).

Das Einbringen eines Moleküls oder eines Konstrukts kann sowohl mittels physikalischer Methoden (wie Mikroinjektion oder Elektroporation), mittels chemischer Methoden (wie mit Kalziumphosphat oder Lipofektion) oder mittels viraler Methoden (durch Viren) erfolgen.

Aus der Beeinflussung der miR-92 Expression in der Zelle folgt die Beeinflussung der Expression eines Proteins in der Zelle, das ausgewählt ist aus der Gruppe bestehend aus: eNOS, SIRT1, Integrin alpha 5, Integrin beta 1, Integrin alpha v, Sprouty 2, TIMP 4, Tie 2, ANG 2, MKK 4, KLF 2, PCAF, EDG 1 und RAP 1B. Alle diese Proteine sind assoziiert mit der Kontrolle der endothelialen Zellfunktion, der Artheroprotektion und/oder der postnatalen Neovaskularisierung. SIRT1 ist zudem mit altersbedingten Erkrankungen assoziiert.

Das Bereitstellen einer Zelle umfasst das Bereitstellen sowohl eine Zelle in isolierter Form, wie beispielsweise einer Zelle einer klonalen Zelllinie, als auch das Bereitstellen einer Zelle, die Teil eines Gewebes, Organs oder eines Organismus ist.

Die bereitgestellte Zelle kann grundsätzlich jede Art von Zelle umfassen. Bevorzugter Weise ist die bereitgestellte Zelle eine vaskuläre Zelle, eine hämatopoetische Zelle, eine Herzmuskelzelle, eine inflammatorische Zelle und/oder eine neuronale Zelle. Dies schließt jeweils sämtliche Vorläufer- und Stammzellen der genannten Zellen ein. In einer bevorzugten Ausführungsform des Verfahrens ist die Stammzelle keine humane embryonale Stammzelle. Weiterhin kann die bereitgestellte Zelle sowohl vereinzelt vorliegen, als auch im Verband eines Gewebes oder eines Organs. In einer Ausführungsform kann das Verfahren auch in vivo durchgeführt werden.

Die bereitgestellte Zelle stammt bevorzugt von einem Metazoen, insbesondere von einem Säugetier (etwa einem Nagetier wie Maus oder Ratte), einem Affen, Menschenaffen, einem Rind, einem Schwein, einem Hund oder einer Katze oder, besonders bevorzugt, von einem Menschen.

### Verringerung der miR-92 Expression

Sofern das Verfahren zu einer Verringerung der miR-92 Expression in der Zelle gegenüber der normalen Expression von miR-92 in einer Wildtyp Zelle führt, erfolgt die Verringerung der Expression durch Bereitstellung eines Moleküls, das ausgewählt ist aus der Gruppe bestehend aus antisense Molekülen, synthetischen miR-92 Inhibitoren und Transkriptionsfaktorinhibitoren. Diese Verringerung der miR-92 Expression hat z. B. eine Verstärkung der Vaskularisierung und Gefäßreparatur im Gewebe zur Folge.

In einer Ausführungsform des erfindungsgemäßen Verfahrens ist das antisense Molekül ein Molekül, dass mit einem RNA-Molekül gemäß einer der SEQ ID NO 1 bis 5 hybridisiert, sowohl unter stringenten als auch unter weniger stringenten Bedingungen. Dies schließt die Möglichkeit mit ein, dass das antisense Molekül im hybridisierten Zustand gegenüber der miR-92 "Mismatches" aufweist, die jedoch die Funktion als antisense Molekül nicht aufheben. Es kommt somit trotz Basenfehlpaarung(en) zur Inhibition oder zum Abbau der miR-92. Insbesondere kann das antisense Molekül eine Länge von bis zu 80 Nukleotiden, bevorzugt bis zu 30 Nukleotiden, besonders bevorzugt von 15 bis 22 Nukleotiden aufweisen. Insbesondere zur Inhibition der unreifen miR-92 Vorläufer kann das antisense Molekül bis zu 80 Nukleotide, insbesondere bis zu 30 Nukleotide aufweisen. Die minimale Länge eines derartigen antisense Molekül beträgt vorteilhafter Weise 12 Nukleotide, bevorzugt 15 Nukleotide.

In einer bevorzugten Ausführungsform weist das antisense Molekül eine Sequenz auf, die zu einer Sequenz gemäß einer der SEQ ID NO 1 bis 5 revers komplementär ist. Dadurch kommt es nicht zur Ausbildung von Basenfehlpaarung(en), was eine effiziente Inhibierung der miR-92 zur Folge hat. In einer bevorzugten Ausgestaltung der Erfindung ist das antisense Molekül ein Molekül mit einer Sequenz gemäß SEQ ID NO 6 oder 8. Es ist möglich, dass das antisense Molekül mindestens eine chemische Modifikationen aufweist, etwa mindestens eine 2' O-Methyl-, Cholestrol-, Phosphothioat- und/oder 2'-O-Methoxyethyl- 2'-Fluoro-Gruppe. Das antisense Molekül kann auch oder zusätzlich locked nucleic acid (LNA) Komponenten aufweisen. Beispielsweise ist eine sehr bevorzugte Ausführungsform des antisense Moleküls mit einer Sequenz gemäß SEQ ID NO 6 mit chemischen Modifikationen das folgende Molekül:
CₜAₜGGCCGGGACAAGUGCAₜAₜUₜAₜ-Chol (SEQ ID NO 9)
   wobei die Großbuchstaben für 2' O-Methyl modifizierte Nukleotide,
   das tiefgestellte t ("ₜ") für eine Phosphothioatbindung zwischen benachbarten Nukleotiden, und
   "Chol" für eine Cholesterolgruppe steht.
Eine sehr bevorzugte Ausführungsform des antisense Moleküls mit einer Sequenz gemäß SEQ ID NO 8 mit chemischen Modifikationen ist das folgende Molekül:
AₜCₜAₜGGCCGGGACAAGUGCAₜAₜUₜAₜ-Chol (SEQ ID NO 10)
   wobei die Großbuchstaben für 2' O-Methyl modifizierte Nukleotide,
   das tiefgestellte t ("ₜ") für eine Phosphothioatbindung zwischen benachbarten Nukleotiden, und
   "Chol" für eine Cholesterolgruppe steht.
Bevorzugt ist ebenfalls ein antisense Molekül mit einer Sequenz gemäß SEQ ID NO 11, das nur 2' O-Methyl modifizierte Nukleotide aufweist.
Verfahren zur Synthese derartiger antisense Moleküle sind dem Fachmann bekannt.

Ein Verfahren der oben beschriebenen Art zur Förderung der Angiogenese, Vaskularisierung und der Gefäßreparatur kann zur Therapie einer Krankheit oder eines Zustands verwendet werden. Die Krankheit oder der (ggf. pathologische) Zustand ist ausgewählt aus der Gruppe bestehend aus Ischämie (wie Myokardinfarkt, chronischer ischämischer Herzkrankheit, periphäre-, oder Herzarterienokklusion, ischämischem Infarkt, Schlaganfall), pathologischer Angiogenese (wie Tumorangiogenese, Metastasenbildung, diabetische Retinopathie, chronischen Entzündungskrankheiten), Artherosklerose, Folgeerkrankungen der Artherosklerose (Akutes Koronarsyndrom, Myokard-Infarkt, Schlaganfall, Kardiomyopathie) oder (vorzeitiger) Alterung oder alterungsassoziierte Erkrankungen.

Gleichfalls kann das beschriebene Verfahren zur Behandlung von Stammzellen oder Vorläuferzellen (wie pro-angiogenen Zellen, organ-spezifischen Vorläuferzellen, aus dem Knochenmark abgeleiteten Zellen oder zirkulierenden Vorläuferzellen) eingesetzt werden.

Somit kann das Verfahren sowohl *in vivo,* als auch *ex vivo* oder *in vitro* eingesetzt werden, etwa zur Herstellung von Gefäßersatzmaterial, insbesondere für die Gewebeersatztherapie, oder in der Forschung.

### Erhöhung der miR-92 Expression

Sofern bei dem Verfahren die Expression der miR-92 in einer Zelle, z. B. in einer Endothelzelle, gegenüber der normalen Expression von miR-92 in einer Wildtyp Zelle erhöht wird, kann dies beispielsweise durch die Erhöhung der Expression mittels Einbringen eines eine exprimierbare miR-92 Sequenz aufweisenden Konstrukts in die Zelle erfolgen. In einer bevorzugten Ausführungsform des erfindungsgemäßen Verfahrens weist das miR-92 Konstrukt eine exprimierbare Sequenz gemäß einer der SEQ ID NO 1 bis 5 auf bzw. kann eine derartige Sequenz exprimieren. Vorteilhafte Ausgestaltungen eines derartigen Konstrukts sind oben beschrieben worden. Alternativ kann auch miR-92 direkt in die Zelle eingebracht werden.

Ein Verfahren der zuletzt beschriebenen Art zur Hemmung der Tumorangiogenese kann zur Therapie einer Krankheit oder eines (pathologischen) Zustands eingesetzt werden, der ausgewählt ist aus der Gruppe bestehend aus überschießender Angiogenese, unerwünschter Angiogenese, Tumoren und chronischen Entzündungen.

### Pharmazeutische Zusammensetzung

Die Offenbarung beschreibt weiterhin eine pharmazeutische Zusammensetzung, aufweisend entweder ein Mittel zur Verringerung der miR-92 Aktivität oder Expression in einer Zelle in Form eines antisense Moleküls gegen miR-92, eines, ggf. synthetischen, Inhibitors und/oder eines Transkriptionsfaktorinhibitors; oder ein Mittel zur Erhöhung der miR-92 Expression in einer Zelle in Form eines Konstrukts zur Expression von miR-92. Dabei kann das Konstrukt eine exprimierbare Sequenz gemäß einer der SEQ ID NO 1 bis 5 aufweisen oder enthalten. Das in der pharmazeutischen Zusammensetzung enthaltend antisense Moleküls gegen miR-92 weist in einer Ausführungsform eine Sequenz gemäß SEQ ID NO 6, 8 oder 11 auf. Für weitere bevorzugte Ausgestaltungen des Konstrukts oder des antisense Moleküls wird auf die oben und hierin enthaltene Beschreibung verwiesen.

Die Offenbarung betrifft weiterhin ein Verfahren zur Herstellung der oben beschriebenen pharmazeutischen Zusammensetzungen.

Die pharmazeutische Zusammensetzung kann in Form von Tabletten, Dragees, Pillen, Granulaten, Aerosolen, Infusionslösungen, Emulsionen, Suspensionen, Lösungen bzw. in oder auf dem Beschichtungsmaterial eines implantierbaren medizinischen Geräts, beispielsweise eines Stents vorliegen.

Die erfindungsgemäße Verwendung der Mittel bzw. der pharmazeutischen Zusammensetzung kann durch geeignete bekannte Formulierungen geschehen.

Die erfindungsgemäße Verwendung der Mittel kann in bekannter Weise in die üblichen Formulierungen überführt werden, wie Tabletten, Dragees, Pillen, Granulate, Aerosole, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Mittelkonzentration in Bezug auf die therapeutisch wirksame Verbindungen jeweils in einer Konzentration von etwa 0,1 Gew.-% bis 95 Gew.-%, bevorzugt von etwa 0,5 Gew.-% bis 90 Gew.-% der Gesamtmischung vorhanden sein, d. h. in Mengen, die ausreichend sind, um die erforderlichen Wirkstoffkonzentrationen im Zielgewebe zu erreichen.

Die Formulierungen werden beispielsweise hergestellt durch Verstrecken der Mittel mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfsstoffe verwendet werden können.

Als Hilfsstoffe erwähnt seien z. B. Wasser, nicht-toxische Lösungsmittel wie Parafin (z. B. Erdölfraktionen), pflanzliche Öle (z. B. Erdnuß-, Sesamöl), Alkohole (z. B. Ethylalkohol, Glycerin), Trägerstoffe, wie natürliche Gesteinsmehle (z. B. Kaoline, Tonerde, Talkum, Kreide), synthetische Gesteinsmehle (z. B. hochdisperse Kieselsäure, Silikate), Zucker (z. B. Rohr-, Milch- und Traubenzucker), Emulgiermittel (z. B. Polyoxyethylen-Fettsäure-Ester, Polyoxyethylen-Fettalkohol-Ether), Dispergiermittel (z. B. Lignin, Sulfitablaugen, Methylcellulose, Stärke und Polyvinylpyrolidon) und Gleitmittel (z. B. Magnesiumstearat, Talkum, Stearinsäure und Natriumsulfat).

Die Applikation erfolgt in üblicher Weise, bevorzugt oral oder parenteral, insbesondere perlingual oder intravenös. Im Falle der oralen Anwendung der erfindungsgemäßen Medikamente können Tabletten selbstverständlich außer den genannten Trägerstoffen auch Zusätze, wie Natriumcitrat, Kalziumcarbonat und Dikalziumphosphat zusammen mit verschiedenen Zuschlagstoffen, wie Stärke, vorzugsweise Kartoffelstärke, Gelatine, und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden. Für den Fall der parenteralen Anwendung können Lösungen der Wirkstoffe unter Verwendung geeigneter flüssiger Trägermaterialien eingesetzt werden.

### Verfahren zur Therapie oder Prophylaxe

Die Offenbarung betrifft ebenfalls ein Verfahren zur Therapie oder Prophylaxe eines Individuums, insbesondere eines Patienten, unter Verwendung eines miR-92 oder eines miR-92 Antagonisten (etwa eines antisense Moleküls oder eines Transkriptionsfaktorinhibitors) oder einer pharmazeutischen Zusammensetzung gemäß der obigen oder hierin enthaltenen Beschreibung. Im Rahmen eines derartigen Verfahren können Wirkstoffmengen zwischen 0,001 mg bis 200 mg pro kg Körpergewicht pro Tag verabreicht.

### Antisense Molekül gegen miR-92

Die Erfindung betrifft auch ein antisense Molekül zur Verwendung gemäß Anspruch 6, gegen miR-92 aufweisend eine Sequenz, die mit einem Molekül einer Sequenz gemäß SEQ ID NO 1 bis 5 hybridisiert, wobei das Molekül bis zu 80 Nukleotide, insbesondere 15 bis 22 Nukleotide aufweist oder daraus besteht. Eines derartigen antisense Molekül weist vorteilhafter Weise mindestens 12 Nukleotide, bevorzugt mindestens 15 Nukleotide auf. Die Hybridisierung kann bei stringenten oder weniger stringenten Bedingungen erfolgen. Verfähren zur Bestimmung der Hybridisierung sind dem Fachmann bekannt.

Ein derartiges Molekül kann ggf. chemische Modifikationen aufweisen. Besonders bevorzugt ist ein Molekül gemäß SEQ ID NO 6, 8 oder 11, das mindestens eine der oben genannten chemischen Modifikationen aufweist. Bevorzugte antisense Moleküle mit chemischen Modifikationen sind die Moleküle gemäß SEQ ID NO 9 und 10, die jeweils von den Molekülen gemäß SEQ ID NO 6 bzw. 8 abgeleitet sind.

Ein derartiges Molekül kann zur Förderung der Angiogenese, Vaskularisierung und der Gefäßreparatur verwendet werden.
miR-92 bzw. ein Molekül mit einer Sequenz nach einem der SEQ ID NO 1 bis 5 kann zur Hemmung der Tumorangiogenese und insbesondere zur Herstellung einer oben beschriebenen pharmazeutischen Zusammensetzung verwendet werden.

Insbesondere in einer bevorzugten Ausführungsform betrifft die Erfindung keine Verfahren zum Klonen von menschlichen Lebewesen oder Verfahren zur Veränderung der genetischen Identität der Keimbahn des menschlichen Lebewesens, sowie nicht die Verwendung von menschlichen Embryonen zu industriellen oder kommerziellen Zwecken oder Verfahren zur Veränderung der genetischen Identität von Tieren, die geeignet sind, Leiden dieser Tiere ohne wesentlichen medizinischen Nutzen für den Menschen oder das Tier zu verursachen, sowie die mit Hilfe solcher Verfahren erzeugten Tiere.

Die Erfindung wird anhand von Beispielen näher erläutert, ohne auf diese Beispiele beschränkt zu sein. Die Ergebnisse der in den Beispielen beschriebenen Versuche sind in den Figuren dargestellt.

### Figuren

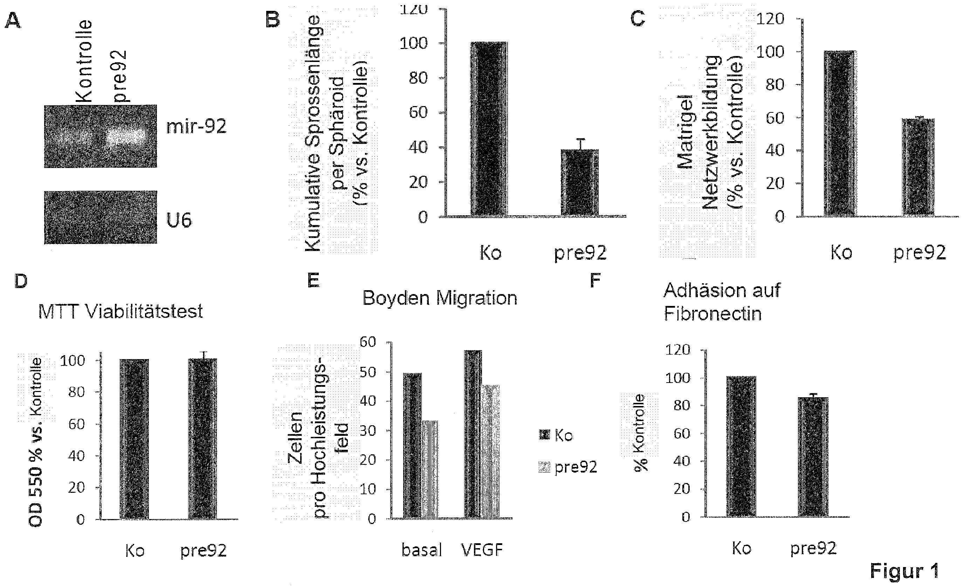

- Es zeigen: Figur 1:: Effekte der Überexpression von miR-92 auf *in vitro* Funktionen von Endothelzellen (als bereitgestellte Zellen).
(A) Überexpression von miR-92 in Prä-miR-92 transfizierten Endothelzellen.
(B/C) Inhibierung der Sprossenbildung in einem Sphäroid (B) und einem "Matrigel Assay" (C).
(D/E/F) Effekt von prä-miR-92 auf die Viabilität (D) Migration (E) und Adhäsion (F).

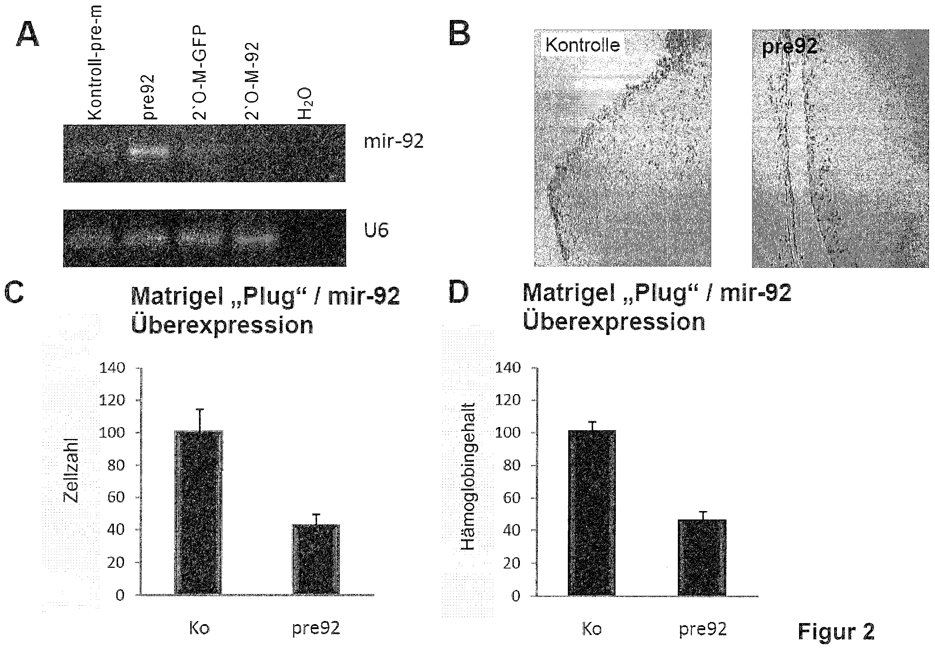

- Figur 2:: Den Effekt der Überexpression von miR-92 auf die *in vivo* Angiogenese.
(A) Überexpression von miR-92 in prä-miR-92 transfizierte Endothelzellen.
(B/C/D)Inhibierung der Angiogenese *in vivo.* HUVEC wurden mit einer Kontrolle oder mit prä-miR-92 transfiziert und 1 x 10⁶ Zellen wurden *in vivo* "Matrigel Plugs" implantiert. Die Angiogenese wurde in H&E-Schnitten (B/C) bestimmt und die Perfusion wurde durch Messung des Hämoglobins bestimmt (D).

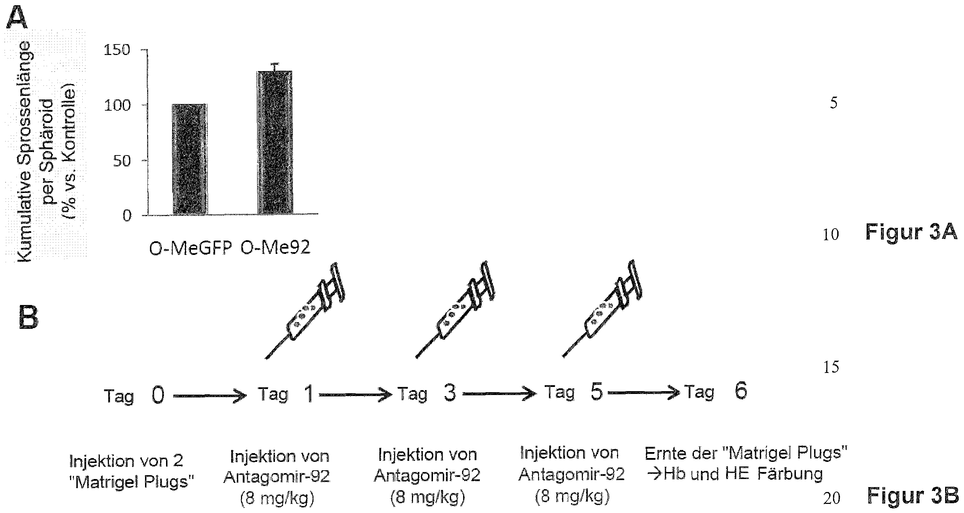

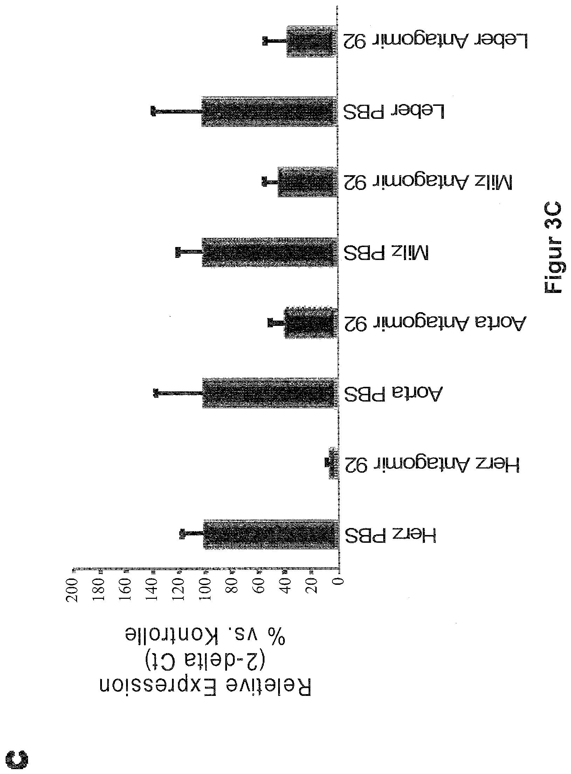

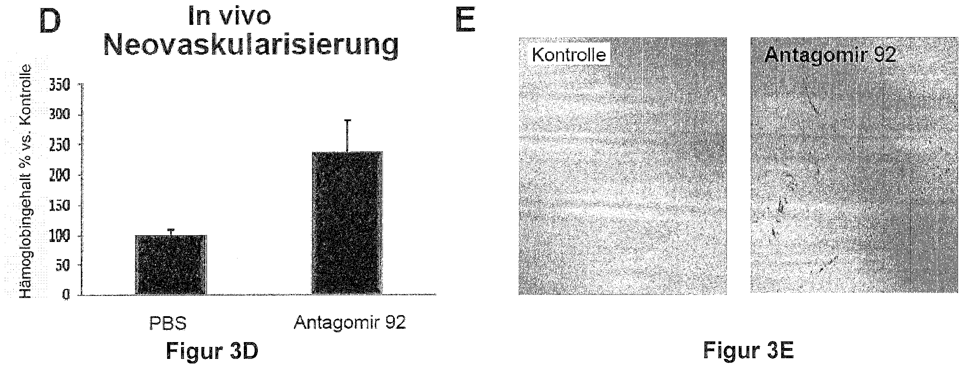

- Figur 3:: Den Effekt der miR-92 Inhibierung auf die *in vitro* und *in vivo* Angiogenese.
(A) Stimulierung der *in vitro* Sprossung mittels 2'O-methyloligonukleotide, die miR-92 blockieren.
(B/C/D/E) Der Effekt der systemischen Fusion eines Antagomirs auf die *in vivo* Neovaskularisierung. (B) experimentelles Design, (C) Expression von miR-92 in verschiedenen Organen (Herz, Aorta, Milz, Leber). (D) Effekte auf die Perfusion gemäß der Messergebnisse der Hämoglobinkonzentration, (E) repräsentative Bilder (H&E-Färbung) zur Darstellung der Gefäßbildung.
- Figur 4:: Die Identifizierung von miR-92 Zielmolekülen.
(A) Genexpressionsprofil HUVEC nach Behandlung mit prä-miR-92.
(B/C) Bestätigung der Dysregulation der Proteinexpression durch Western Blot in HUVEC nach Transfektion von prä-miR-92.
(D/E) Detektion des Effekts von prä-miR-92 auf die Integrinexpression mittels FACS.

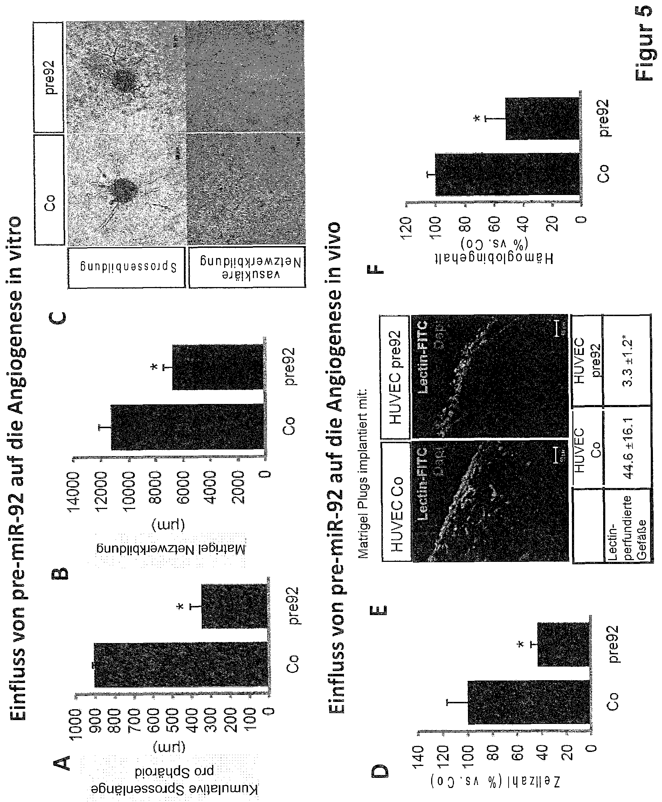

- Figur 5:: Einfluss von miR-92a auf die Angiogenese in vitro (a-c) und in vivo (d-f). In vitro: Pre-miR-92 wurde in HUVEC überexprimiert und die Angiogenese im Spheroidmodel (a) und Matrigelassay (b) in vitro bestimmt. N>3, *p<0.05 versus Kontrolle (Co). Panel C zeigt repräsentative Beispiele. In vivo: Pre-miR-92a oder KontrollmicroRNA (Co) transfizierte HUVEC wurden mit Matrigel gemischt und in Nacktmäuse transplantiert. Die Gefäßbildung wurde durch Nachweis der eingewanderten Zellen (d), Bestimmung der Lektin in vivo perfundierten Gefäße (e)und durch Nachweis des Hämoglobingehalts (f) bestimmt. N>4, *p<0.05 versus Kontrolle (Co).

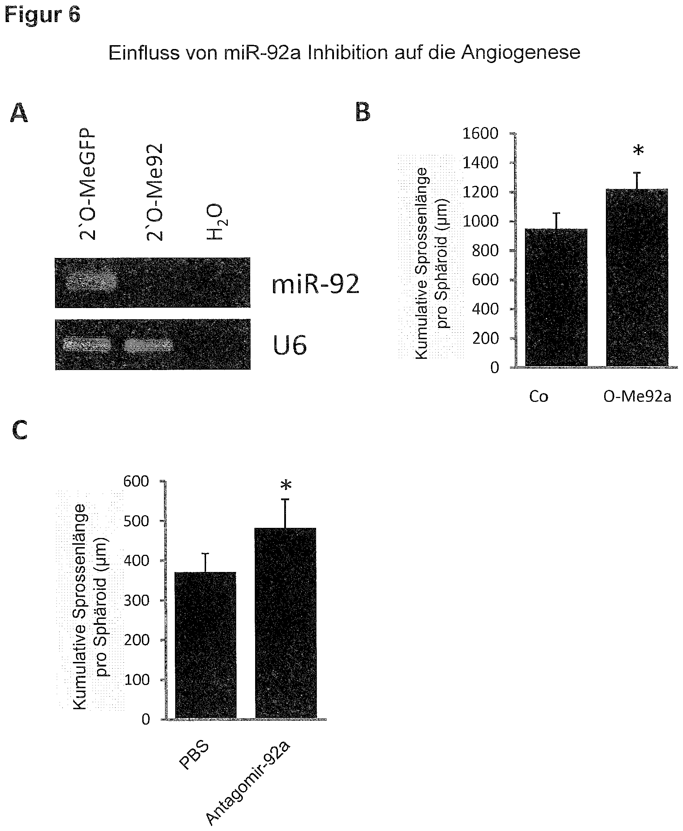

- Figur 6:: Einfluss von miR-92a Inhibition auf die Angiogenese in vitro.
a/b) miR-92a wurde durch Überexpression von 2'O-Methyl antisense Oligoribonukleotiden blockiert und die Angiogenese im Spheroidmodel in vitro bestimmt. N>3, *p<0.05 versus Kontrolloligonukleotide (Co) (2'OMeGFP).
c) miR-92a wurde durch die Inkubation mit Antagomir-92a inhibiert und die Angiogenese im Spheroidmodel in vitro bestimmt. N=5, *p<0.05 versus PBS Kontrolle.

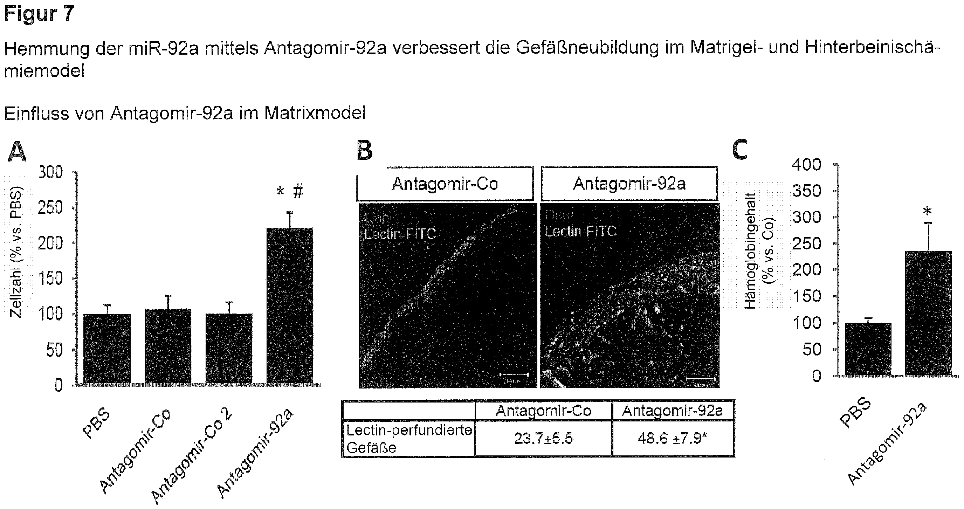

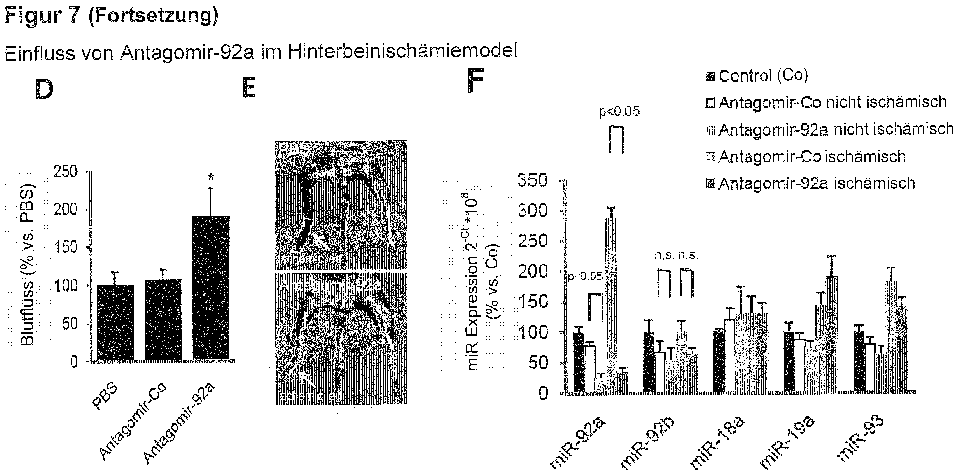

- Figur 7:: Einfluss von Antagomir-92a auf die Gefäßneubildung im Matrigelmodel (a-c) und Hinterbeinischämiemodel (d-f).
Antagomir-92a, 2 verschiedene Kontrollantagomirs oder das Lösungsmittel PBS wurden an Tag 1, 3, 5 i.v. injiziert (8 mg/kg bw) und die Matrigelplugs an Tag 7 explantiert. Die Anzahl der eingewanderten Zellen (H&E, a), perfundierten Lectinpositiven Gefäßen (b) und der Hämoglobingehalt (c) wurden bestimmt. N>4, p<0.05 versus PBS oder Antagomir-Kontrollen (Co). Antagomir-92a oder Kontrollen wurden an Tag 0, 2, 4, 7, 9 nach Hinterbeinischämie i.v. injiziert und die Durchblutung mittels Laserdoppler bestimmt (d, Beispiel E). Abbildung F zeigt die Spezifität der Antagomir-92a auf die Expression von miR-92a und verschiedenen anderen microRNAs. N>3, p<0.05 versus Kontrollen (Co).

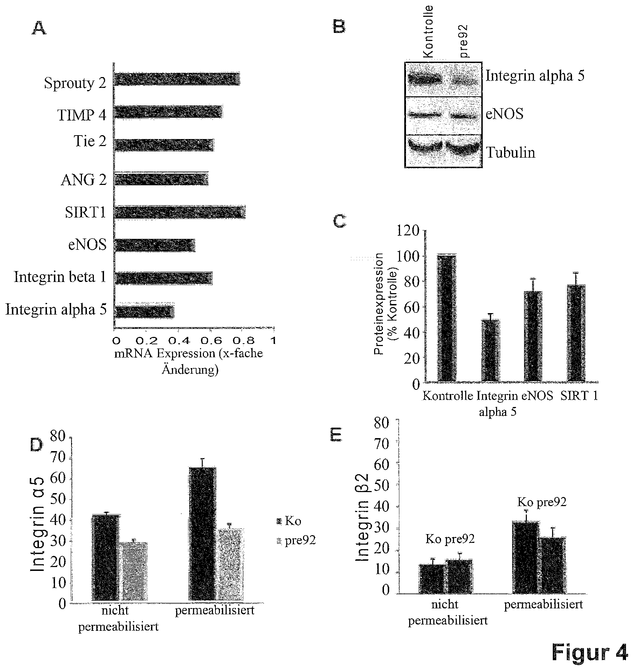

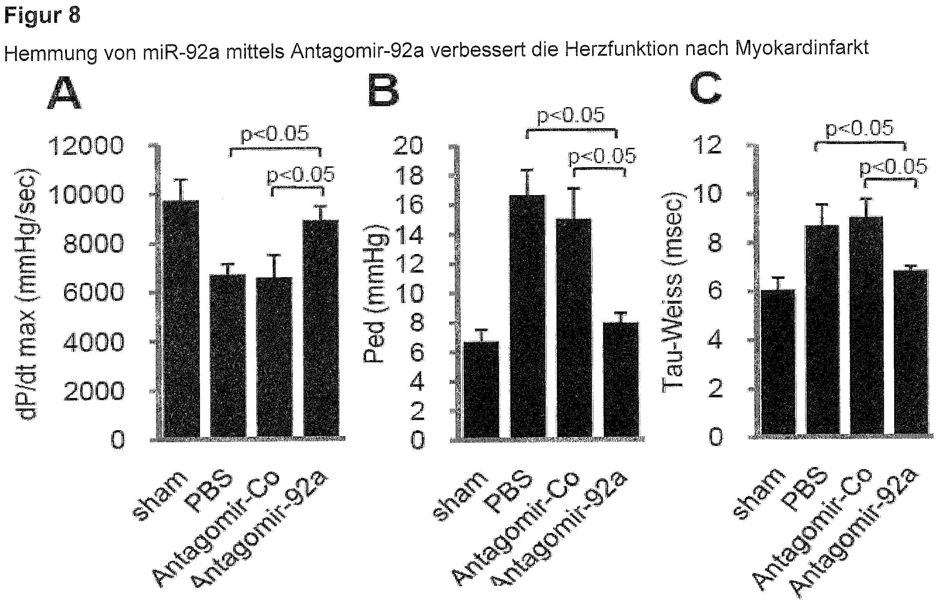

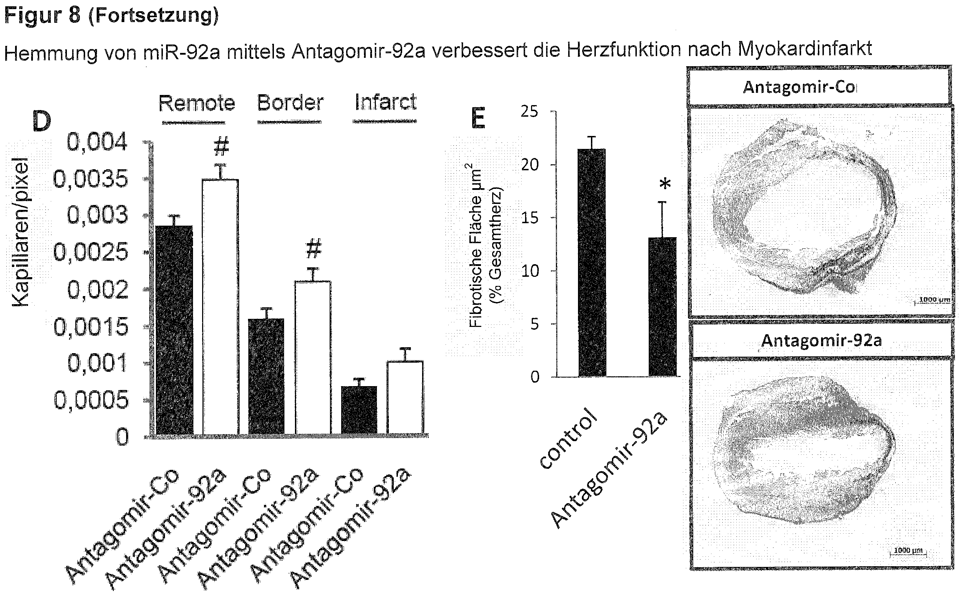

- Figur 8:: Abbildung 4: Einfluss von Antagomir-92a auf die Herzfunktion nach Myokardinfarkt. Antagomir-92a, Kontrollantagomir (Antagomir-Co) oder das Lösungsmittel PBS wurden nach Induktion des Herzinfarkts an Tag 0, 2, 4, 7, 9 i.v. injiziert (8 mg/kg bw) und die Herzfunktion an Tag 14 mittels Millar Katheter bestimmt. a-c zeigt die Bestimmung von Herzfunktionsparametern (a: Kontraktilität, b: Druck, c: Relaxationskonstante). Antagomir-92a behandelte Tiere zeigen durchwegs bessere Herzfunktionsparameter im Vergleich zu der Antagomir-Co und PBS Gruppe. D) zeigt den signifikanten Anstieg der Kapillaren in verschiedenen Regionen des Herzens nach Antagomir-92a Behandlung. E) zeigt die Reduktion der Infarktgrösse und Fibrosierung in der Antagomir-92a behandelten Gruppe. Alle Experimente N>5, *p<0.05 versus Kontrolle (Co).

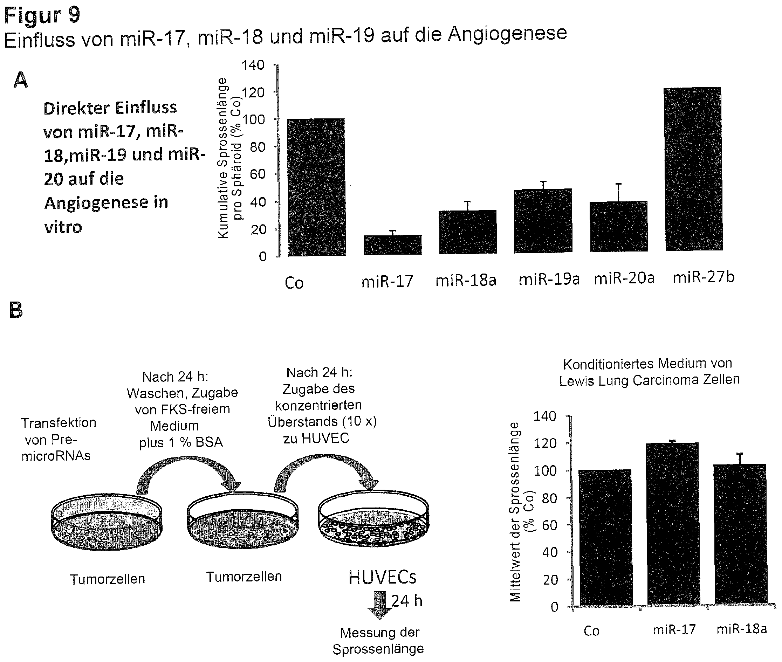

- Figur 9:: Einfluss der einzelnen Mitglieder des miR-17-92 Clusters auf die Angiogenese in vitro.
a) Direkter Effekt der Überexpression der einzelnen MicroRNAs auf die Angiogenese im Spheroid Assay (N>3, *p<0.05).
b) Parakriner Effekt von Tumorzellen, die mit miR-17 oder miR-18 transfiziert wurden auf die Angiogenese von Endothelzellen. Wie dargestellt wurden Tumorzellen transfiziert und dann der Überstand auf Endothelzellen (HUVEC) gegeben, um die Wirkung von parakrinen Faktoren zu testen. Die Überexpression von miR-17 führt zu einem Anstieg der Angiogenese.

### Beispiele

### Material und Methoden

### Zellkultur

Menschliche Nabelschnurvenen-Endothelzellen (HUVEC) wurden von Cambrex erworben und in endothelialem Basalmedium (EBM; Cambrex) ergänzt mit Hydrokortison, Rinderhirnextrakt, epidermalem Wachstumsfaktor und 10 % fötalem Kälberserum (FCS; Gibco) bis zur dritten Passage kultiviert. Nach dem Ablösen mit Trypsin wurden die Zellen in 6 cm Kulturschalen für mindestens 24 bis 48 Stunden kultiviert.

### Transfektion

Zur Inhibition von miR-92 wurden HUVEC vor der Transfektion mit dem spezifischen Inhibitor bis zu einer Konfluenz von 60 % bis 70 % kultiviert. 2'O-methylantisens Oligoribonukleotide gegen miR-92 (5'- CAGGCCGGGACAAGUGCAAUA -3' SEQ ID NO 11) oder GFP (5'- AAGGCAAGCUGACCCUGAAGUU -3', SEQ ID NO 7) wurden von VBC Biotech synthetisiert und 50 nmol/l wurden mit GeneTrans II^{®} (MoBiTec) gemäß dem Protokoll des Herstellers transfiziert. Zur Überexpression von miR-92 wurden HUVEC bis zu einer Konfluenz von 50 % kultiviert. 10 nmol/l prä-miR-92 oder Kontroll-prä-miR (Ambion) wurden mit Lipofectamin RNAiMAX (Invitrogen) gemäß dem Protokoll des Herstellers transfiziert.

### Antagomir-Strategie

Die hier verwendete einzelsträngige RNA bestand aus 21 bis 23 Nukleotiden und wurde von VBC Biotech wie beschrieben (13) synthetisiert. Alle Tiermodelle wurden in einem C57BL/6J Hintergrund gehalten. Acht Wochen alte Mäuse wurden subkutan entlang der abdominalen Mittellinie mit zwei "Matrigel Basement Matrix Plugs" am Tag 0 injiziert und erhielten Schwanzveneninjektionen einer Salzlösung oder eines Antagomirs 92 am Tag 1,3 und 5. Antagomir 92 wurde in Dosen von 8 mg pro kg Körpergewicht in 0,2 ml Phosphat gepufferte Saline (PBS) pro Injektion verabreicht. Gewebe und "Matrigel Plugs" wurden am sechsten Tag geerntet. Das Gewebe wurde in flüssigem Stickstoff eingefroren und zur RNA Analyse bei -80 °C aufbewahrt. Zur Hämoglobinanalyse wurde ein "Matrigel Plug" nach sieben Tagen entfernt und in 130 µl deionisiertem Wasser homogenisiert. Nach der Zentrifugation wurde der Überstand in einem Drabkin Assay (Sigma-Aldrich) zur Messung der Hämoglobinkonzentration verwendet. Stammlösungen des Hämoglobins wurden zur Herstellung einer Standardkurve verwendet. Die Ergebnisse werden relativ zum Gesamtprotein in Überstand ausgedrückt. Der zweite "Matrigel Plug" wurde zur Quantifizierung eindringender Zellen mittels H&E-Färbung verwendet.

### Western Blot Analyse

Zur Western Blot Analyse wurden HUVEC in RIPA Lyse Puffer (Sigma) für 20 Minuten auf Eis lysiert. Nach einer Zentrifugation für 15 Minuten bei 20.000 x g (4 °C) wurde der Proteingehalt der Proben nach der Bradford Methode bestimmt. Gleiche Proteinmengen wurden auf ein SDS-Polyacrylamidgel geladen und auf eine PVDF oder Nitrozellulosemembran geblottet. Western Blots wurden unter Verwendung von Antikörpern gegen Integrin a5 (Kaninchen polyklonaler Anti-Integrin a5 Antikörper; 1:250, Chemicon), MKK4 (Kaninchen polyklonaler Anti-MKK4, 1:1.000, Cell Signaling), eNOS (Maus monoklonaler Anti-eNOS, 1:2.500, BD), SIRT1 (Kaninchen polyklonaler Anti-SIRT1, 1:1.000, Upstate) oder Tubulin (Maus monoklonaler Anti-Tubulin; 1:1.500, Dianova) durchgeführt.

### RT-PCR

Um die differenzielle miRNA Expression in HUVEC zu bestimmen, die mit 2'O-methylantisens Oligoribonukleotiden gegen miR-92 oder Prä-miR-92 transfiziert wurden, wurde 24 Stunden nach der Transfektion Gesamt RNA unter Verwendung von TRIzol (Invitrogen) gemäß dem Protokoll des Herstellers isoliert. Eine RT-PCR wurde unter Verwendung des *mir*Vana™ qRT-PCR miRNA Detektionskit (Ambion) und Primersets durchgeführt, die zur Amplifikation von hsa-miR-92 (Ambion) spezifisch sind (ein Zyklus: 3 Minuten bei 95 °C, 25 Zyklen: 15 Sekunden bei 95 °C, 30 Sekunden bei 60 °C).

### Migrationsassay

Um die Migration von Endothelzellen zu bestimmen, wurden HUVEC mit Trypsin abgelöst, durch Zentrifugation geerntet und in 500 µl EBM mit 0,1 BSA resuspendiert, gezählt und in die obere Kammer einer modifizierten Boydenkammer (5 x 10⁴ Zellen pro Kammer, Porengrößen 8 µm, BD Biosciences) platziert, die mit 2,5 µg/l Fibronektin beschichtet war. Die Kammer wurde in eine Kulturschale mit 24 Vertiefungen platziert, welche EBM mit 0,1 % BSA und menschlichem vaskulären endothelialem Wachstumsfaktor (VEGF, 50 ng/ml) enthielten. Nach einer Inkubation für 5 Stunden bei 37 °C wurden die nicht migrierenden Zellen auf der oberen Seite der Kammer mechanisch entfernt und die verbleibenden Zellen auf der unteren Seite wurden mit 4 % Formaldehyd fixiert. Zur Quantifizierung wurden die Zellkerne mit 4',6-Diaminophenylidol (DABI) gefärbt. Migrierende Zellen auf der unteren Seite der Kammer wurden in fünf zufällig gewählten mikroskopischen Feldern manuell gezählt.

### Gefäßbildungsassay

HUVEC (7 x 10⁴) wurden in einer Platte mit 12 Vertiefungen (Greiner) beschichtet mit 200 µl "Matrigel Basement membrane Matrix" (BD Biosciences) kultiviert. Die gebildeten endothelialen Netzwerke wurden nach 24 Stunden in fünf zufällig gewählten mikroskopischen Feldern mittels eines computergesteuerten Mikroskops unter Verwendung des Programms KS300 3.0 (Zeiss) quantifiziert.

### Spheroid basierter Angiogenese Assay

Endotheliale Zellsphäroide von definierter Zellzahl wurden wie beschrieben hergestellt (22, 23). Die *in vitro* Angiogenese wurde durch Messung der kumulativen Länge der gesprossenen Strukturen ermittelt, die aus jedem Spheroid gewachsen waren, und zwar unter Verwendung einer digitalen Bildsoftware (Axioplan, Zeiss), wobei 10 Sphäroide pro experimenteller Gruppe und Experiment analysiert wurden.

### MTT Viabilitätsassay

Die Messung der Zellviabilität erfolgte unter Verwendung des (3-(3,4-Dimethylthiazol-2-yl)-2,5-diphenyl-2H-tetrazoliumbromid) (MTT Assays). 48 Stunden nach der Transfektion wurden 0,5 mg/ml MTT in jede Vertiefung gegeben und die Zellen wurden für 4 Stunden bei 37 °C inkubiert. Die Zellen wurden mit PBS gewaschen und 30 Minuten bei Raumtemperatur mit Lysepuffer (40 nmol/l HCl in Isopropanol) lysiert. Die Absorption wurde bei 550 nm fotometrisch gemessen.

### Zellmatrix Adhäsion

Zellkulturplatten mit 96 Vertiefungen wurden über Nacht bei 4 °C mit 1 µg/ml löslichem rekombinantem humanem Kollagen I (Roche, Mannheim, Deutschland) oder 2,5 µg/ml humanem Fibronektin (Roche, Mannheim, Deutschland) in PBS beschichtet und dann für ein Stunde bei Raumtemperatur mit 3 % (w/v) Hitze-inaktiviertem (2 Stunden, 56 °C) humanem Serumalbumin (HSA) inkubiert. HUVEC wurden mit 2',7'-Bis-(2-carboxyethyl)-5-(und-6)-carboxyfluoresceinacetoxymethylester (BCECF-AM) oder CellTracker Green (Molecular Probes, Eugene, Oregon) gefärbt und nach der Ablösung mit Trypsin in EBM mit 0,05 % HSA resuspendiert. Dann wurden 50.000 Zellen pro Vertiefung in 100 µl EBM mit 0,05 % HSA in die beschichteten Vertiefungen ausgesät und für 60 Minuten bei 37 °C inkubiert. Nach dem Auswaschen nicht-adhärierender Zellen mit warmem EBM wurden die adhärenten Zellen dreifach mit einem Fluoreszenzplattenlesegerät (Fluostat, BMG Lab Technologies, Offenburg, Deutschland) quantifiziert.

### Durchflusszytometrieanalyse

Zur Permeabilisierung wurden mit prä-miR-92 oder Kontrolle transfizierte HUVEC mit Trypsin abgelöst, in 4 % Formaldehyd für 10 Minuten fixiert und mit 0,1 % TritonX-100 behandelt. Die Zellen (permeabilisiert und nicht-permeabilisiert) wurden unter Verwendung von 1 % BSA blockiert und mit Integrin a5 (Anti-CD49e-FITC 1:10, Immunotech) oder Integrin b1 (Anti-CD29-APC 1:20, BD) Antikörpern gefärbt. Die Zellen wurden mit einem FACS Canto II Gerät (BD) analysiert.

### In vivo "Matrigel Plug" Assay

Dieser Assay wurde wie beschrieben (24) durchgeführt, jedoch mit den folgenden Modifikationen:
HUVEC wurden mit prä-miR-92 oder zur Kontrolle wie oben beschrieben transfiziert. 18 Stunden nach der Transfektion wurden die Zellen mit "cell tracker CM-Dil" (Invitrogen) markiert, abgelöst, gewaschen und gezählt. 1 x 10⁶ Zellen wurden in 30 µl PBS resuspendiert und mit 500 µl "Matrigel Basement Membrane Matrix" (BD Biosciences) 15 Einheiten Heparin (Sigma-Aldrich) enthaltend, gemischt. Die Zell-Matrigel-Mischung wurde in sechs bis acht Wochen alte weibliche athymische Nackt-Mäuse (Harlan) entlang der abdominalen Mittellinie subkutan injiziert. Hämoglobinanalyse and H&E-Färbung wurden wie oben beschrieben durchgeführt.

### Affymetrix mRNA Proriling

HUVECs wurden mit prä-miR-92 oder zur Kontrolle transfiziert. Gesamt RNA wurde nach 48 Stunden isoliert und das Genexpressionsprofil wurde mittels eines Affymetrix-Gen-Chip-Expressionsassays gemessen

### Ergebnisse

### Prä-miR-92 blockiert endotheliale Zellfunktionen in vitro und in vivo

Um den Effekt von miR-92 auf Endothelzellen zu testen, wurden HUVEC mit dem miR-92 Vorläufer prä-miR-92 transfiziert und der Effekt dieser Transfektion in verschiedenen *in vitro* Assays bestimmt. Die effiziente Überexpression von miR-92 wurde zunächst mittels RT-PCR nachgewiesen (Figur 1A). Die miR-92 Überexpression blockierte signifikant die Bildung von Gefäßstrukturen in einem Sphäroidassay (Figur 1B) und inhibierte die Bildung eines vaskulären Netzwerkes in Matrigelen (Figur 1C), was dafür spricht, dass miR-92 ein negativer Regulator der Angiogenese *in vitro* ist. Um einen möglicherweise toxischen Effekt von miR-92 zu ermitteln, wurde die Zellviabilität gemessen. Dabei konnten nach Transfektion von prä-miR-92 im Vergleich zu nicht-transfizierten Zellen keine signifikanten Unterschiede ermittelt werden (Figur 1D). Da die endotheliale Zellmigration für die Angiogeneseaktivität von Zellen *in vitro* von großer Bedeutung ist, wurde zusätzlich die Migration von HUVEC unter basalen Bedingungen und als Antwort auf VEGF bestimmt. Prä-miR-92 verringerte die Migration (Figur 1E) und die Adhäsion der Zellen auf Fibronektin (Figur 1F). Daher zeigt prä-mir-92 keinen direkten toxischen Effekt, sondern blockiert die endotheliale Zellantwort, die für Angiogenese benötigt wird. Weiterhin wurde der Effekt von prä-miR-92 auf die Angiogenese *in vivo* bestimmt. Dazu wurden mit prä-miR-92 transfizierte HUVEC in einen "Matrigel Plug" in Nacktmäuse *in vivo* implantiert. Die Effizienz der Inhibierung wurde jeweils in einer Subfraktion der implantierten Zellen kontrolliert (Figur 2A). Wie in den repräsentativen Bildern der Figur 2B und der Quantifizierung in Figur 2C gezeigt ist, blockiert prä-miR-92 das Wachstum der Gefäße *in vivo* in effizienter Weise. Außerdem ist die Perfusion signifikant reduziert, was durch Messung der Hämoglobinkonzentration in den explantierten "Matrigel Plugs" nachgewiesen werden konnte (Figur 2B).

### Inhibierung von miR-92 erhöht die Angiogenese in vitro und in vivo

Weiterhin wurde untersucht, ob die Inhibierung von miR-92 die Stimulation des Gefäßwachstums zur Folge hat. MiR-92 wurde durch 2'-O-methyl antisens Oligoribonukleotide (O-methyl-miR-92) inhibiert und die Bildung von Gefäßstrukturen *in vitro* wurde mittels eines Spheroidassays bestimmt. O-methyl-miR-92 erhöhte die Sprossenbildung *in vitro* (Figur 3A), was dafür spricht, dass die Inhibierung von miR-92 eine neue therapeutische Strategie zur Verbesserung der Angiogenese darstellen könnte. Um diese Hypothese zu testen, wurde miR-92 unter Verwendung so genannter "Antagomirs", einzelsträngige RNA Oligonukleotide, die gegenüber spezifischen miRNAs eine komplementäre Sequenz aufweisen, systemisch inhibiert. Chemische Modifikationen führen zu einer erhöhten Stabilität und Cholesterol-Konjugation zu einer verbesserten Aufnahme in die Zellen (15). Gegen miR-92 gerichtete Antagomirs wurden wie in Figur 3B dargestellt an drei Tagen verabreicht. Die systemische Gabe von Antagomirs verbesserte das Gefäßwachstum und die Perfusion des "Matrigel Plugs" *in vivo* (Figur 3C/D). Im Ergebnis erhöht die Inhibierung von miR-92 endotheliale Zellfunktionen *in vitro* und verbessert das Gefäßwachstum *in vivo.*

### Identifizierung von miR-92 Ziel-Genen

miRNAs kontrollieren Zielgene durch Abbau der Ziel-mRNA oder durch translationale Repressionen. Um die Ziel mRNAs bestimmen zu können, die als Antwort auf die miR-92 Überexpression abgebaut werden, wurde eine Chipanalyse mit einem Affymetrix mRNA Genexpressionsarray mit 54.681 Genen (HG-U133 Plus 2) durchgeführt. Die Analyse der regulierten mRNAs identifizierte verschiedene Schlüsselenzyme, die die endotheliale Funktion kontrollieren, einschließlich eNOS, SIRT1, Integrine und Wachstumsfaktoren wie Angeopoietin-2 (Figur 4A). Ein Teil der herunterregulierten Gene sind mit einer *in silico* durchgeführten Analyse potentieller miR-92 Ziele kompatibel (Tabelle 1). Um die Ergebnisse des Screens zu bestätigen, wurde die Proteinexpression der jeweiligen Gene durch Western Blot oder FACS Analyse detektiert. Im Einklang mit den vorhergesagten Ergebnissen wurde die Proteinexpression von eNOS, SIRT1 und Integrin a5 durch prä-miR-92 signifikant unterdrückt (Figur 4B bis E).

**Tabelle 1: In silico Vorhersage von miR-92 Zielmolekülen, über die die biologische Wirkung von miR-92 vermittelt wird**

| **miR-92 - Ziele** |
|---|
| Integrin a5 |
| Integrin av |
| SIRT 1 |
| MKK4 |
| KLF 2 |
| PCAF |
| EDG 1 |
| RAP 1B |

Im Ergebnis zeigt miR-92 einen starken anti-angiogenen Effekt und beeinflusst endotheliale Zellfunktionen *in vitro* und *in vivo* negativ. In Übereinstimmung damit führt die Blockade von miR-92 durch systemische Infusion eines Antagomirs zu einem verbesserten Gefäßwachstum *in vivo.* Dieses Ergebnis ist überraschend, da es im Gegensatz zur beschriebenen pro-angiogenen Aktivität des miR-17-92 Clusters steht (10).

Die vorliegenden Daten zeigen, dass miR-92 die Expression verschiedener Proteine beeinflusst, die dafür bekannt sind, eine tragende Rolle in der endothelialen Zellbiologie zu spielen. Unter den mittels eines Mikroarrays identifizieren Genen konnte insbesondere die Herunterregulation von eNOS, SIRT1 und Integrin a5 auf Proteinebene bestätigt werden. Mäuse, die für diese Proteine defizient sind, zeigen eine beeinträchtigte vaskulären Funktion und/oder eine beeinträchtigte Fähigkeit zur postnatalen Neovaskularisierung.

eNOS spielt eine Rolle in der Aufrechterhaltung der Vasoreaktivität und blockiert Apoptose endothelialer Zellen (16). Die Histondeacetylase SIRT1 fördert die Langlebigkeit in Modellorganismen und kontrolliert die Neovaskularisierung und Gefäßreifung in Säugern (13, 17). Die Dysregulation von Integrinen kann sich negativ auf die Interaktion mit der Zellmatrix auswirken und somit anti-apoptotische Signalgebung und Zellmigration beeinträchtigen (14, 18). Der Wachstumsfaktor Angiopoietin-2, sein Rezeptor Tie2 und die Proteaseinhibitoren wie TIMP4 kontrollieren die Gefäßreifung (19, 20). Demzufolge interagiert miR-92 mit einer Reihe von Genen, die endotheliale Zellfunktionen auf verschiedenen Ebenen kontrollieren. Die Fähigkeit von miR-92 verschiedene Effektoren zu beeinflussen, stellt einen Vorteil der miRNA-basierten therapeutischen Strategie dar und trägt dazu bei, über die beschränkte therapeutische Kapazität einer auf einem einzigen Wachstumsfaktor oder einem einzigen Gen basierenden Therapie einer ischämischen Krankheit hinwegzukommen, da die komplexen Prozesse des Gefäßwachstums, der Gefäßreifung und der funktionalen Erhaltung der Gefäße bekannter Weise eine fein abgestimmte Regulation einer Reihe von Genen voraussetzen.

Im Ergebnis stellt die Beeinflussung von miR-92 eine neuartige therapeutische Strategie zur Kontrolle endothelialer Zellfunktionen dar. Insbesondere die hier gezeigte systemische Verwendung von Antagomirs ist zur Beeinflussung der miRNA Funktionen geeignet. Die Inhibierung von miR-92 durch Antagomirs erhöht das Gefäßwachstum und trägt zur Verbesserung der Neovaskularisierung und der Gefäßreparation bei. In Bezug auf die Gene, die für ihre artheroprotektive Wirkung bekannt sind, wie beispielsweise eNOS, ist die Blockade von miR-92 in der antiartherosklerotischen Therapie von Nutzen. Da SIRT 1 auch eine neuroprotektive Rolle in neurodegenerativen Erkrankungen (z. B. Parkinson) zugeschrieben wird (25), können miR-92 Antagomirs auch in diesem Krankheitsbild erfolgreich eingesetzt werden. Im Gegensatz dazu ist die Überexpression von miR-92 beispielsweise zur Blockade der Tumorangiogenese nützlich, da durch sie das Gefäßwachstum stark verringert wird.

Insbesondere umfasst die vorliegende Erfindung die folgenden Ausführungsformen:
Ausführungsform 1: Verfahren zur Beeinflussung der miR-92 Expression in einer Zelle, aufweisend die folgenden Schritte:
   - Bereitstellen einer Zelle; und
   - Verringern der miR-92 Expression in der Zelle zur Förderung der Vaskularisierung oder Gefäßreparatur durch Einbringen eines antisense Moleküls gegen miR-92 in die Zelle, oder
   - Erhöhen der miR-92 Expression in der Zelle zur Hemmung der Tumorangiogenese durch Einbringen eines Konstrukts in die Zelle, wobei das Konstrukt eine exprimierbare miR-92 Sequenz aufweist.
Ausführungsform 2: Verfahren nach Ausführungsform 1, wobei die bereitgestellte Zelle von einem Metazoen, insbesondere von einem Säugetier stammt.
Ausführungsform 3: Verfahren nach Ausführungsform 1 oder 2, wobei die bereitgestellte Zelle eine vaskuläre Zelle, eine hämatopoetische Zelle, eine Herzmuskelzelle, eine inflammatorische Zelle, eine neuronale Zelle, eine Vorläuferzelle oder eine Stammzelle ist, wobei die Stammzelle keine humane embryonale Stammzelle ist.
Ausführungsform 4: Verfahren nach Ausführungsform 1 bis 3, wobei das antisense Molekül ein Molekül ist, dass mit einem RNA-Molekül gemäß einer der SEQ ID NO 1 bis 5 hybridisiert, insbesondere ein Molekül mit einer Sequenz gemäß SEQ ID NO 6, 8 oder 11.
Ausführungsform 5: Verfahren nach Ausführungsform 1 bis 4, wobei das antisense Molekül eine Sequenz aufweist, die zu einer Sequenz gemäß einer der SEQ ID NO 1 bis 5 komplementär ist.
Ausführungsform 6: Verfahren nach Ausführungsform 1 bis 5, wobei das antisense Molekül eine Länge von bis zu 30 Nukleotiden, insbesondere von 15 bis 22 Nukleotiden aufweist.
Ausführungsform 7: Verfahren nach Ausführungsform 1 bis 6, wobei die exprimierbare miR-92 Sequenz eine Sequenz gemäß SEQ ID NO 1 bis 5 aufweist.
Ausführungsform 8: Verwendung eines Verfahrens nach Ausführungsform 1 bis 6 zur Therapie einer Krankheit oder eines Zustands, der ausgewählt ist aus der Gruppe bestehend aus Ischämie, pathologische Angiogenese, Artherosklerose, Folgeerkrankungen der Artherosklerose und Alterungs-assoziierten Krankheiten, wobei die zelluläre miR-92 Expression durch Einbringen eines antisense Moleküls gegen miR-92 in die Zelle verringert wird.
Ausführungsform 9: Verwendung eines Verfahrens nach Ausführungsform 1 bis 3 oder 7, zur Therapie einer Krankheit oder eines Zustands, wobei die Krankheit oder der Zustand ausgewählt ist aus der Gruppe bestehend aus überschießender Angiogenese, unerwünschter Angiogenese, Tumoren und chronischen Entzündungen, wobei die zelluläre miR-92 Expression durch Einbringen eines Konstrukts in die Zelle erhöht wird, wobei das Konstrukt eine exprimierbare miR-92 Sequenz aufweist.
Ausführungsform 10: Verwendung eines Verfahrens nach Ausführungsform 1 bis 6 zur Herstellung von Gefäßersatzmaterial.
Ausführungsform 11: Pharmazeutische Zusammensetzung, aufweisend
   - ein Mittel zur Verringerung der miR-92 Aktivität oder Expression in einer Zelle in Form eines antisense Moleküls gegen miR-92, oder
   - ein Mittel zur Erhöhung der miR-92 Expression in einer Zelle in Form eines Konstrukts zur Expression von miR-92.
Ausführungsform 12: Pharmazeutische Zusammensetzung nach Ausführungsform 11, wobei das Konstrukt eine exprimierbare Sequenz gemäß einem der SEQ ID NO 1 bis 5 aufweist.
Ausführungsform 13: Pharmazeutische Zusammensetzung nach Ausführungsform 11, wobei das antisense Moleküls gegen miR-92 eine Sequenz gemäß SEQ ID NO 6, 8 oder 11 aufweist.
Ausführungsform 14: Antisense Molekül aufweisend eine Sequenz, die mit einem Molekül einer Sequenz gemäß SEQ ID NO 1 bis 5 hybridisiert, wobei das antisense Molekül bis zu 30 Nukleotide, insbesondere zwischen 15 und 22 Nukleotide aufweist, insbesondere ein antisense Molekül mit einer Sequenz gemäß SEQ ID NO 6, 8 oder 11.
Ausführungsform 15: Verwendung eines Moleküls nach Ausführungsform 14 zur Verringerung der miR-92 Aktivität oder miR-92 Expression in einer Zelle.
Ausführungsform 16: Verwendung von miR-92 zur Förderung der Vaskularisierung und der Gefäßreparatur oder zur Hemmung der Tumorangiogenese.
Ausführungsform 17: Verwendung von miR-92, insbesondere zur Herstellung einer pharmazeutischen Zusammensetzung, zur Behandlung von Ischämie, pathologischer Angiogenese, Artherosklerose, Folgeerkrankungen der Artherosklerose oder Alterungs-assoziierte Krankheiten.

### Referenzen

1 Carmeliet, P. (2000) Mechanisms of angiogenesis and arteriogenesis. Nat Med 6 (4), 389-395.
2 Adams, R.H. and Alitalo, K. (2007) Molecular regulation of angiogenesis and lymphangiogenesis. Nat Rev Mol Cell Biol 8 (6), 464-478
3 Bartel, D.P. (2004) MicroRNAs: genomics, biogenesis, mechanism, and function. Cell 116 (2), 281-297
4 Hofacker, I.L. (2007) How microRNAs choose their targets. Nat Genet 39 (10), 1191-1192
5 Yang, W.J. et al. (2005) Dicer is required for embryonic angiogenesis during mouse development. J Biol Chem 280 (10), 9330-9335
6 Suarez, Y. et al. (2007) Dicer dependent microRNAs regulate gene expression and functions in human endothelial cells. Circ Res 100 (8), 1164-1173
7 Kuehbacher, A. et al. (2007) Role of Dicer and Drosha for endothelial microRNA expression and angiogenesis. Circ Res 101 (1), 59-68
8 Poliseno, L. et al. (2006) MicroRNAs modulate the angiogenic properties of HUVECs. Blood 108 (9), 3068-3071
9 Venturini, L. et al. (2007) Expression of the miR-17-92 polycistron in chronic myeloid leukemia (CML) CD34+ cells. Blood 109 (10), 4399-4405
10 Dews, M. et al. (2006) Augmentation of tumor angiogenesis by a Myc-activated microRNA cluster. Nat Genet 38 (9), 1060-1065
11 Murohara, T. et al. (1998) Nitric oxide synthase modulates angiogenesis in response to tissue ischemia. J Clin Invest 101 (11), 2567-2578
12 Aicher, A. et al. (2003) Essential role of endothelial nitric oxide synthase for mobilization of stem and progenitor cells. Nat Med 9 (11), 1370-1376
13 Potente, M. et al. (2007) SIRT1 controls endothelial angiogenic functions during vascular growth. Genes Dev 21 (20), 2644-2658
14 Kim, S. et al. (2000) Regulation of integrin alpha vbeta 3-mediated endothelial cell migration and angiogenesis by integrin alpha5betal and protein kinase A. J Biol Chem 275 (43), 33920-33928.
15 Krutzfeldt, J. et al. (2005) Silencing of microRNAs in vivo with 'antagomirs'. Nature 438 (7068), 685-689
16 Dimmeler, S. and Zeiher, A.M. (1999) Nitric oxide - an endothelial cell survival factor. Cell Death Differ 6, 964-968
17 Imai, S. et al. (2000) Transcriptional silencing and longevity protein Sir2 is an NADdependent histone deacetylase. Nature 403 (6771), 795-800.
18 Zhang, Z. et al. (1995) The alpha 5 beta 1 integrin supports survival of cells on fibronectin and up-regulates Bcl-2 expression. Proc Natl Acad Sci U S A 92 (13), 6161-6165
19 Asahara, T. et al. (1998) Tie2 receptor ligands, Angiopoietin-1 and Angiopoietin-2, modulate VEGF-induced postnatal neovascularization. Circ Res 83, 233-240
20 Sang, Q.X. (1998) Complex role of matrix metalloproteinases in angiogenesis. Cell Res 8 (3), 171-177
21 Care, A. et al. (2007) MicroRNA-133 controls cardiac hypertrophy. Nat Med 13 (5), 613-618
22 Korff, T. and Augustin, H.G. (1998) Integration of endothelial cells in multicellular spheroids prevents apoptosis and induces differentiation. J Cell Biol 143 (5), 1341-1352
23 Diehl, F. et al. (2007) The histone methyltransferase MLL is an upstream regulator of endothelial cell sprout formation. Blood 109 (4), 1472-1478
24 Potente, M. et al. (2005) Involvement of FoxO transcription factors in angiogenesis and postnatal neovascularization. J Clin Invest 115(9), 2382-2392.
25 Dillin, A. and Kelly, J.W. (2007) The yin-yang of sirtuins. Science 317 (5837), 461-462

## Patentansprüche

1. Verwendung eines antisense Moleküls zur Herstellung einer pharmazeutischen Zusammensetzung zur Behandlung oder zur Vorbeugung des akuten Koronarsyndroms, Myokard-Infarkts, Schlaganfalls, Kardiomyopathie, chronisch ischämische Herzkrankheit, periphere Arterienokklusion, oder ischämischer Infarkt, wobei das antisense Molekül komplementär zu miR-92 ist.

2. Die Verwendung nach Anspruch 1, wobei das antisense Molekül eine Länge von bis zu 30 Nukleotiden, insbesondere von 15 bis 22 Nukleotide aufweist.

3. Die Verwendung nach einem der vorstehenden Ansprüche, wobei das antisense Molekül zumindest eine chemische Modifikation umfasst, wobei die zumindest eine chemische Modifikation eine 2'-O-Methylgruppe, eine Cholesterolgruppe, eine Phosphorthioat-Gruppe, eine 2'-O-Metoxyethyl-Crruppe, eine 2'-Fluor-Gruppe, oder eine locked nucleic acid (LNA) Komponente ist.

4. Die Verwendung nach einem der vorstehenden Ansprüche, wobei das antisense Molekül eine Sequenz umfasst, die komplimentär ist zu einem Molekül der Sequenzen der SEQ ID Nr. 1, 2, 3, 4 oder 5.

5. Verwendung nach einem der vorstehenden Ansprüche, wobei das antisense Molekül eine Sequenz gemäß SEQ ID Nr. 6, 8, oder 11 umfasst.

6. Ein antisense Molekül zur Verwendung in der Behandlung oder Vorbeugung des akuten Koronarsyndroms, Myokard-Infarkts, Schlaganfalls, Kardiomyopathie, chronisch ischämische Herzkrankheit, periphere Arterienokklusion, oder ischämischer Infarkt, wobei das antisense Molekül komplementär zu miR-92 ist.

7. Das antisense Molekül zur Verwendung gemäß Anspruch 6, wobei das antisense Molekül eine Länge von bis zu 30 Nukleotiden, insbesondere von 15 bis 22 Nukleotide aufweist.

8. Das antisense Molekül zur Verwendung gemäß der Ansprüche 6 oder 7, wobei das antisense Molekül mindestens eine chemische Modifikation umfasst, wobei die zumindest eine chemische Modifikation eine 2'-O-Methylgruppe, eine Cholesterolgruppe, eine Phosphorthioat-Gruppe, eine 2'-O-Metoxyethyl-Gruppe, eine 2'-Fluor-Gruppe, oder eine locked nucleic acid (LNA) Komponente ist.

9. Das antisense Molekül zur Verwendung gemäß der Ansprüche 6 bis 8, wobei das antisense Molekül eine Sequenz umfasst, die komplimentär ist zu einem Molekül einer Sequenz der SEQ ID Nr. 1, 2, 3, 4, oder 5.

10. Das antisense Molekül zur Verwendung gemäß der Ansprüche 6 bis 9, wobei das antisense Molekül eine Sequenz gemäß der SEQ ID Nr. 6, 8, oder 11 umfasst.

## Claims

1. Use of an antisense molecule for producing a pharmaceutical composition for the treatment or prevention of acute coronary syndrome, myocardial infarct, stroke, cardiomyopathy, chronic ischemic heart disease, peripheral artery occlusion, or ischemic infarct, wherein the antisense molecule is complementary to miR-92.

2. The use of according to claim 1, wherein the antisense molecule has a length of up to 30 nucleotides, in particular, between 15 and 22 nucleotides.

3. The use of any one of the preceding claims, wherein the antisense molecule comprises at least one chemical modification, wherein the at least one chemical modification is a 2'-O-methyl-group, cholesterol-group, phosphorothioate-group, 2'-O-methoxyethyl-group, 2'-fluoro-group, or locked nucleic acid (LNA) component.

4. The use of any one of the preceding claims, wherein the antisense molecule comprises a sequence that is complementary to a molecule of a sequence of SEQ ID NO 1, 2, 3, 4 or 5.

5. The use of any one of the preceding claims, wherein the antisense molecule comprises a sequence according to SEQ ID NO 6, 8 or 11.

6. An antisense molecule for use in treatment or prevention of acute coronary syndrome, myocardial infarct, stroke, cardiomyopathy, chronic ischemic heart disease, peripheral artery occlusion, or ischemic infarct, wherein the antisense molecule is complementary to miR-92.

7. The antisense molecule for use according to claim 6, wherein the antisense molecule has up to 30 nucleotides, in particular, between 15 and 22 nucleotides.

8. The antisense molecule for use according to claim 6 or 7, wherein the antisense molecule comprises at least one chemical modification, wherein the at least one chemical modification is a 2'-O-methyl-group, cholesterol-group, phosphorothioate-group, 2'-O-methoxyethyl-group, 2'-fluoro-group, or locked nucleic acid (LNA) component.

9. The antisense molecule for use according to any one of claims 6 to 8, wherein the antisense molecule comprises a sequence that is complementary to a molecule of a sequence of SEQ ID NO 1, 2, 3, 4 or 5.

10. The antisense molecule for use according to any one of claims 6 to9, wherein the antisense molecule comprises a sequence according to SEQ ID NO 6, 8 or 11.

## Revendications

1. Utilisation d'une molécule antisens pour la préparation d'une composition pharmaceutique destinée au traitement ou à la prévention du syndrome coronarien aigu, de l'infarctus du myocarde, de l'attaque cérébrale, de la cardiomyopathie, de la cardiopathie ischémique chronique, de l'artériopathie périphérique oblitérante ou de l'infarctus ischémique, la molécule antisens étant complémentaire à miR-92.

2. Utilisation selon la revendication 1, dans laquelle la molécule antisens présente une longueur allant jusqu'à 30 nucléotides, notamment de 15 à 22 nucléotides.

3. Utilisation selon l'une des revendications précédentes, dans laquelle la molécule antisens comprend au moins une modification chimique, qui est un groupe 2'-O-méthyle, un groupe cholestérol, un groupe phosphorothioate, un groupe 2'-O-méthoxyéthyle, un groupe 2'-fluoro, ou un composant à acide nucléique bloqué (LNA).

4. Utilisation selon l'une des revendications précédentes, dans laquelle la molécule antisens comprend une séquence qui est complémentaire à une molécule des séquences de SÉQ. ID N°1, 2, 3, 4 ou 5.

5. Utilisation selon l'une des revendications précédentes, dans laquelle la molécule antisens comprend une séquence selon la SÉQ. ID N°6, 8, ou 11.

6. Molécule antisens pour une utilisation dans le traitement ou la prévention du syndrome coronarien aigu, de l'infarctus du myocarde, de l'attaque cérébrale, de la cardiomyopathie, de la cardiopathie ischémique chronique, de l'artériopathie périphérique oblitérante ou de l'infarctus ischémique, la molécule antisens étant complémentaire à miR-92.

7. Molécule antisens pour l'utilisation selon la revendication 6, ladite molécule antisens présentant une longueur pouvant aller jusqu'à 30 nucléotides, en particulier de 15 à 22 nucléotides.

8. Molécule antisens pour l'utilisation selon les revendications 6 ou 7, ladite molécule antisens comprenant au moins une modification chimique, qui est un groupe 2'-O-méthyle, un groupe cholestérol, un groupe phosphorothioate, un groupe 2'-O-méthoxyéthyle, un groupe 2'-fluoro, ou un composant à acide nucléique bloqué (LNA).

9. Molécule antisens pour l'utilisation selon les revendications 6 à 8, ladite molécule antisens comprenant une séquence qui est complémentaire à une molécule d'une séquence des SÉQ. ID N°1, 2, 3, 4 ou 5.

10. Molécule antisens pour l'utilisation selon les revendications 6 à 9, ladite molécule antisens comprenant une séquence selon la SÉQ. ID N°6, 8, ou 11.


### SEQUENCE LISTING

<110> t2cure GmbH
<120> Verfahren zur Förderung der Angiogenese, Vaskularisierung oder Gefäßreparatur oder zur Hemmung der Tumorangiogenese
<130> FB22960/A
<150> DE 10 2007 052 114.8
   <151> 2007-10-30
<160> 11
<170> PatentIn version 3.3
<210> 1
   <211> 78
   <212> RNA
   <213> Homo sapiens
<400> 1
   cuuucuacac agguugggau cgguugcaau gcuguguuuc uguaugguau ugcacuuguc      60
   ccggccuguu gaguuugg                                                    78
<210> 2
   <211> 75
   <212> RNA
   <213> Homo sapiens
<400> 2
   ucaucccugg guggggauuu guugcauuac uuguguucua uauaaaguau ugcacuuguc 60
   ccggccugug gaaga 75
<210> 3
   <211> 96
   <212> RNA
   <213> Homo sapiens
<400> 3
   cgggccccgg gcgggcggga gggacgggac gcggugcagu guuguuuuuu cccccgccaa 60
   uauugcacuc gucccggccu ccggcccccc cggccc 96
<210> 4
   <211> 22
   <212> RNA
   <213> Homo sapiens
<400> 4
   uauugcacuu gucccggccu gu 22
<210> 5
   <211> 22
   <212> RNA
   <213> Homo sapiens
<400> 5
   uauugcacuc gucccggccu cc 22
<210> 6
   <211> 21
   <212> RNA
   <213> artificial
<220>
   <223> Antisense Oligoribonukleotid gegen miR-92
<400> 6
   caggccggga caagugcaau a 21
<210> 7
   <211> 22
   <212> RNA
   <213> artificial
<220>
   <223> 2'O-methyl-antisense Oligoribonukleotid gegen GFP
<220>
   <221> misc_feature
   <222> (1)..(22)
   <223> 2'0-methyl modifiziertes Nukleotid
<400> 7
   aaggcaagcu gacccugaag uu 22
<210> 8
   <211> 22
   <212> RNA
   <213> Artificial
<220>
   <223> Antisense Oligoribonukleotid gegen miR-92
<400> 8
   acaggccggg acaagugcaa ua 22
<210> 9
   <211> 21
   <212> RNA
   <213> Artificial
<220>
   <223> 2\2220-methyl modifiziertes antisense Oligoribonukleotid gegen miR-92
<220>
   <221> misc_feature
   <222> (1)..(2)
   <223> Phosphothioatbindung
<220>
   <221> misc_feature
   <222> (2)..(3)
   <223> Phosphothioatbindung
<220>
   <221> misc_feature
   <222> (18)..(19)
   <223> Phosphothioatbindung
<220>
   <221> misc_feature
   <222> (19) .. (20)
   <223> Phosphothioatbindung
<220>
   <221> misc_feature
   <222> (20)..(21)
   <223> Phosphothioatbindung
<220>
   <221> misc_feature
   <222> (21) .. (21)
   <223> 3' Cholesterolgruppe
<220>
   <221> misc_feature
   <222> (21) .. (21)
   <223> Phosphothioat
<400> 9
   caggccggga caagugcaau a 21
<210> 10
   <211> 22
   <212> RNA
   <213> Artificial
<220>
   <223> 2\2220-methyl modifiziertes antisense Oligoribonukleotid gegen miR-92
<220>
   <221> misc_feature
   <222> (1)..(2)
   <223> Phosphothioatbindung
<220>
   <221> misc_feature
   <222> (2)..(3)
   <223> Phosphothioatbindung
<220>
   <221> misc_feature
   <222> (3)..(4)
   <223> Phosphothioatbindung
<220>
   <221> misc_feature
   <222> (19) .. (20)
   <223> Phosphothioatbindung
<220>
   <221> misc_feature
   <222> (20)..(21)
   <223> Phosphothioatbindung
<220>
   <221> misc_feature
   <222> (21) .. (22)
   <223> Phosphothioatbindung
<220>
   <221> misc_feature
   <222> (22)..(22)
   <223> 3' Cholesterolgruppe
<220>
   <221> misc_feature
   <222> (22)..(22)
   <223> Phosphothioat
<400> 10
   acaggccggg acaagugcaa ua 22
<210> 11
   <211> 21
   <212> RNA
   <213> Artificial
<220>
   <223> 2\2220-methyl modifiziertes antisense Oligoribonukleotid gegen miR-92
<220>
   <221> misc_feature
   <222> (1)..(21)
   <223> 2'O-methyl modifiziertes Nukleotid
<400> 11
   caggccggga caagugcaau a 21